# EUROPÄISCHE PATENTSCHRIFT

(11) **EP 0 980 379 B1**
(45) Veröffentlichungstag und Bekanntmachung des Hinweises auf die Patenterteilung: **07.08.2002**
(21) Anmeldenummer: 98933431.3
(22) Anmeldetag: 04.05.1998
(51) Int. Cl.: C07H 21/00

(54) **CHIMÄRE OLIGONUCLEOTIDE UND IHRE VERWENDUNG**
CHIMERIC OLIGONUCLEOTIDES AND THE USE THEREOF
OLIGONUCLEOTIDES CHIMERIQUES ET LEUR UTILISATION

(30) Priorität: 02.05.1997 DE 19720151
(43) Veröffentlichungstag der Anmeldung: 23.02.2000
(73) Patentinhaber: MAX-DELBRÜCK-CENTRUM FÜR MOLEKULARE MEDIZIN, 13125 Berlin (DE)
(72) Erfinder: MATTHES, Eckart, D-15345 Eggersdorf (DE); VON JANTA-LIPINSKI, Martin, D-12487 Berlin (DE)
(74) Vertreter: Baumbach, Friedrich, Dr.
(86) Internationale Anmeldenummer: DE9801216
(87) Internationale Veröffentlichungsnummer: WO9850397

(56) Entgegenhaltungen:
- EP-A- 0 355 031
- WO-A-96/23508
- WO-A-97/37691
- WO-A-97/38013
- WO-A-98/28442
- US-A- 4 963 662
- US-A- 5 489 508

## Beschreibung

Die Erfindung betrifft neue chimäre Oligonucleotide und ihre Verwendung, insbesondere zur Hemmung des Enzyms Telomerase und zur Herstellung von pharmazeutische Formulierungen, die in der Antitumortherapie Verwendung finden. Somit betrifft die Erfindungen auch Arzneimittel, die die chimären Oligonucleotide enthalten.

Die heutige klinisch angewandte Krebschemotherapie muß als völlig unzureichend angesehen werden. Sie führt nur bei wenigen Tumoren zu einer Heilung, die überwiegende Zahl der bösartigen Tumoren muß mit der derzeitigen Therapie als nicht heilbar angesehen werden. Sie ist hauptsächlich eine unspezifische antiproliferative Therapie, also darauf gerichtet, das Wachstum und die Zellteilung zu hemmen. Dieser Effekt bleibt nicht auf Tumorzellen beschränkt, sondern betrifft auch eine Reihe von Erneuerungsgeweben mit einer hohen Teilungsrate, wie z. B. das Knochenmark, Darm- und Hautepithelzellen, woraus sich die starken cytotoxischen Nebenwirkungen erklären.

Die Erkenntnisse, daß Mutationen in einer Vielzahl von Onkogenen und Repressorgenen der Zellen ursächlich mit der Tumorentstehung verknüpft sind, haben zu einer Vielzahl von Bemühungen um selektive, Ursachen- orientierte Chemotherapeutika geführt.
Dazu gehören z. B. Farnesyltransferase- und Tyrosin-Kinase Inhibitoren, Gentherapien zur Wiederherstellung von Suppressorgenfunktionen bzw. der DNA Reparatur oder Antisenseoligonucleotide gegen verschie-dene Onkogene (z. B. Ras, Raf, Erb). Zu diesen neuen, eine höhere Selektivität und Wirksamkeit versprechenden Krebstargets ist auch die Telomerase zu rechnen.

Die Telomerase ist eine RNA-abhängige DNA Polymerase, die DNA an den 3'-Enden der Chromosomen verlängert. Dabei benutzt sie einen kleinen Abschnitt der mit ihr fest verknüpften RNA als Template für die Synthese einer immer wiederkehrenden Sequenz. Diese sogenannte Telomer-DNA hat beim Menschen die Sequenz TTAGGG.

Ihre Funktion besteht zum einen im Schutz der Chromosomenenden vor Abbau oder Fusion- wodurch karyotypische Veränderungen und genetische Instabilitäten verhindert werden, zum anderen in der Kontrolle der abgelaufenen Zellteilungen. Hiermit in Zusammenhang steht der Befund, daß die Häufigkeit dieser Wiederholungen in verschiedenen Zellen sehr variieren und zwischen etwa 1000 und 12000 liegen kann (Harley, 1991).

Diese Heterogenität der Telomerlänge erklärt sich im wesentlichen durch zwei Regulationselemente. Zum einen durch einen Replikations-bedingten Verlust von Telomer-DNA bei jeder DNA Synthese und damit bei jeder Zellteilung, zum anderen durch die Aktivität der Telomerase, die diesen Verlust in bestimmten Zellen und unter bestimmten Bedingungen ausgleichen kann.
Ohne Kompensationsmöglichkeit erreicht die Telomer DNA Länge jeder sich teilenden Zelle schließlich eine kritische untere Grenze (etwa 7000 Bp; Bacchetti, 1996), die offenbar ein Signal für die Zelle darstellt, ihre Teilungsfähigkeit einzustellen und Alterungsvorgänge einzuleiten. Demnach ist die Länge der Telomereinheiten ein Maß, an dem eine somatische Körperzelle die Anzahl der durchlaufenen Teilungen registrieren kann.

So ist erklärlich, daß die Länge der Telomer DNA in den meisten somatischen Zellen mit dem Alter stark abfällt. Nur die sog. immortalisierten Zellen, zu denen z. B. Keimzellen und fetale Zellen gehören, exprimieren dieses Enzym, die damit über einen Mechanismus verfügen, ver-lorene Telomereinheiten zu ersetzen und den Zellen damit eine nahezu unbegrenzte Teilungs-fähigkeit zu erhalten (Harley, 1991). 1994 wurden erstmals Telomerase-Aktivitäten sowohl in Tumorzellen eines menschlichen Ovarial-Carcinoms als auch in kultivierten menschlichen Tumorzellinien nachgewiesen (Counter et al., 1994 ). Seit dieser Entdeckung gehen die positiven Nachweise der Telomerase in menschlichen Tumoren in die Tausende. Das ist hauptsächlich durch die Entwicklung eines Assays (Telomere Repeat Amplification Protocol, TRAP) möglich geworden, der durch PCR-Amplifikation des Telomeraseproduktes die Empfindlichkeit des Testes auf etwa das 10⁴-fache gesteigert hat und die Bestimmung der Te-lomeraseaktivität in wenigen Zellen (etwa 50 bis 100) möglich gemacht hat (Kim et al., 1994; Piatyszek et al. 1995).

In Abhängigkeit von der Art und dem Stadium der untersuchten Tumoren konnte in 80- 95% von ihnen eine Telomerase-Aktivität nachgewiesen werden (Healy, 1995; Autexier et al., 1996 ; Shay et al., 1996; Hiyama et al., 1997). Offenbar wird das unbegrenzte Proliferationspotential von Tumorzellen zum überwiegenden Teil durch die Expression der Telomerase erreicht, so daß das Wachstum der meisten Tumoren von der Funktion der Telomerase abhängig ist.

Damit kann man die Telomerase als essentiell für das unbegrenzte Teilungsvermögen der meisten Tumorzellen ansehen, womit dieses Enzym zu einem neuen wichtigen Target für eine Krebstherapie geworden ist (Healy, 1995; Holt et al., 1996; Hamilton et al.,1996).

Neben Tumorzellen exprimieren auch Keimzellen als unsterbliche Zellen dieses Enzym. Außerdem sind in den letzten beiden Jahren auch in Stammzellen von Erneuerungsgeweben (z. B. der Haut, des Darmes und des Knochenmarkes) aber auch in Leukozyten und Lymphozyten unter Aktivierungsbedingungen geringe, aber eindeutige Telomeraseaktivitäten gefunden worden (Counter et al., 1995).
Es ist bisher nicht klar, welche Bedeutung die geringen Telomeraseaktivitäten in diesen somatischen Zellen haben und ob die Telomerase hier tatsächlich ständig zur Telomerverlängerung benutzt wird.

Entscheidende Nebenwirkungen sollte eine solche auf die Hemmung der Telomeraseaktivität gerichtete Therapie nicht haben, ausgenommen sind dabei allerdings die menschlichen Keimzellen. Das Proliferationsvermögen der erwähnten Stammzellen mit ihrer vergleichsweise geringen Teilungsrate und dem damit verbundenen geringen Telomerverlust sollte selbst bei einer Hemmung der nur intermittierend auftretenden Telomerase kaum Schaden nehmen (Holt et al., 1996). Damit kann eine solche Antitelomerase-Therapie als eine wirksame und selektive Therapie bösartiger Tumoren angesehen werden, die der herkömmlichen Chemotherapie überlegen ist.

Einige modifizierte Nucleosidtriphosphate sind als mögliche Hemmstoffe der menschlichen Telomerase untersucht worden. Dabei handelt es sich um einige Verbindungen, die vor einigen Jahren als Hemmstoffe der Reversen Transcriptase von HIV (Human Immunodeficiency Virus) entwickelt wurden. Aufgrund der sehr ähnlichen Funktion dieses Enzymes mit der Telomerase, die ja beide eine RNA als Template für eine Synthese von DNA benutzen, haben sich 2',3'-Didesoxyguanosintriphosphat (ddGTP), Guaninarabinosyltriphosphat (araGTP), 2',3'-Didesoxythymidintriphosphat (ddTTP), 2',3'-Didehydro-2',3'-didesoxythymidintriphosphat (ddeTTP) und 3'-Azidothymidintriphosphat (AzTTTP) auch als Hemmstoffe der Telomerase erwiesen.

In ihrer Nucleosidform haben aber nur Azidothymidin (AzT) und 2',3'-Didesoxyguanosin (ddG) nach längere Applikation bei einigen Zellinien zu einer Verkürzung der Telomer DNA geführt, ohne daß dadurch jedoch ihr Wachstumsverhalten entscheidend verändert oder gar einen Proliferationsstopp ausgelöst wurde (Strahl et al., 1996 ).

Beschrieben ist ferner, daß ein anderes therapeutisches Target die mit der Telomerase fest verknüpfte RNA sein könnte. So läßt sich mit Antisense-Oligonucleotiden, die komplementär an bestimmte Regionen der RNA binden seine Templatefunktion blockieren und die Enzymaktivität hemmen.
So konnte in HeLa-Zellen die Funktion der Telomerase durch die Expression eines Antisense Oligonucleotides gegen die Telomerase RNA permanent gehemmt werden und damit eine zunehmende Verkürzung der Telomer DNA erreicht werden, die schließlich nach 23-26 Teilungszyklen zum Zelltod führte (Feng et al., 1995).
Mit modifizierten Antisense- Oligonucleotiden, deren Phosphatzuckergerüst durch das nicht ionische N-(2-Aminoethyl)glycine ersetzt wurde (Peptid-Nucleinsäuren; PNA) konnte die Telomerase in vitro im Nanomolarbereich gehemmt werden (Norton et al., 1996). Auch hier wurde ein Sequenzabschnitt der Telomerase RNA als therapeutisches Target gewählt, der als Template für die Telomer DNA- Synthese benutzt wird. Allerdings ist von diesen excellent bindenden PNA's auch bekannt, daß sie über Zellmembranen prinzipiell nicht aufgenommen werden können und daher an Zellen bisher auch nicht anwendbar sind (Hanvey et al., 1992). In der zierten Arbeitvon Norton et al. sind auch Phosphorthioat-modifizierte Oligonucleotide als hochwirksame aber unspezifische Hemmstoffe der Telomerase beschrieben worden.

Oligomere, die eine sequenzspezifische Wechselwirkung mit der Telomerase zeigen, sind aus WO-A 96/23508 und US-A 5,489,508 bekannt. So beschreibt WO-A 96/23508 synthetische Oligonucleotide, die neoplastische Zellen hemmen und/oder abtöten, wobei die Oligonucleotide eine "single repeat" Sequenz besitzen, die ein telomerisches Repeat darstellt. US-A 5,489,508 beschreibt ein Verfahren und Zusammensetzungen, die zur Bestimmung der Telomerlänge und Telomeraseaktivität geeignet sind.

Es muß jedoch festgestellt werden, daß es zum gegenwärtigen Zeitpunkt keinen an Tumorzellen anwendbaren selektiven und wirksamen Hemmstoff der Telomerase gibt.

Der Erfindung lag deshalb die Aufgabe zugrunde, selektive und hochwirksame Hemmstoffe der menschliche Telomerase zu entwickeln, die dieses Enzym auch in Zellen über einen langen Zeitraum selektiv hemmen zu können.

Die Erfindung wird gemäß den Ansprüchen realisiert und beruht auf der überraschenden Erkenntnis, daß die beschriebenen Phosphothioate nicht an der RNA, sondern sequenzunspezifisch an der Proteinkomponente und zwar an einer sogenannten Primer-Binding Site binden, an der normalerweise die zu verlängernde Telomer DNA zusätzlich zu der Template Region der RNA fixiert ist. Es existieren also zwei Targets an der Telomerase.

Erfindungsgemäß werden diese zwei Targets - 1. die RNA und 2. die Primer Binding Site des Proteins - mit Oligonudeotiden blockiert und damit wird eine therapeutisch optimale Hemmung möglich.

Es wurden erfindungsgemäß Chimäre Oligonucleotide hergestellt, die aus unterschiedlich modifizierten und zwar auf die beiden Targets hin optimierten Oligomeren bestehen, und die beide Bindungsorte der Telomer DNA an dem Enzym gleichzeitig blockieren. Diese zwei unterschiedlich modifizierten Oligonucleotide sind miteinander verknüpft.

Sie haben sich als wirksamer und selektiver erwiesen als ihre Einzelkomponenten. Insbesondere haben sich solche chimären Oligonucleotide bewährt, die am 5'-Ende des Oligonucleotides durch Phosphorthioate modifiziert ist und sich damit an das Protein binden, während sie zum 3'- Ende hin z.B. durch Phosphoramidate substituiert bzw. ggf. über einen Linker mit PNA verknüpft sind, deren Basensequenzen komplementär zur Telomerase RNA sind und damit hochselektiv an die RNA binden sowie die Wirksamkeit der Phosphorthioate wesentlich erhöhen. Darüber hinaus haben wir gefunden, daß eine weitere, erhebliche Wirkungssteigerung erreicht werden kann, wenn das 3'-Ende der erfindungsgemäßen chimären Oligonucleotide durch solche Nucleoside modifiziert ist, die zusätzlich das kaktalytische Zentrum des Enzyms hemmen (z. B. 3'-Azidodesoxyguanosin).

Die erfindungsgemäßen Chimären Oligonucleotide der allgemeinen Formel I sind durch folgende Strukturen gekennzeichnet:

Die erfindungsgemäßen Verbindungen weisen ein Phosphorthioat modifiziertes Oligonucleotid beliebiger Sequenz am 5'-Ende auf und ein anderweitig modifiziertes Oligomer am 3'-Ende, dessen Basensequenz komplementär zur Telomerase RNA ist.

### Formel I ist eine Kombination aus den Formeln II und III/1, III/2 und III/3 (R)

Formel II stellt das Oligomer dar, das an der Primer Binding Site der Telomerase bindet und Formel III stellt das Oligomer dar, das an der RNA bindet.

### Formel II

5'-Ende des chimären Oligonucleotides mit hoher Bindungsfähigkeit an das Protein: 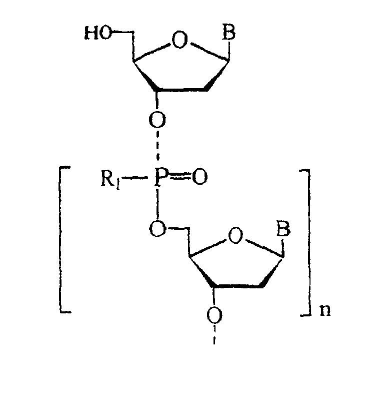 n> 10; ≤ 20
R₁= S⁻, CH₃, O⁻
B= Thymin, Cytosin, Adenin, Guanin

### Formel III/1, III/2 und III/3

3'-Ende des chimären Oligonucleotides mit hoher Bindungsfähigkeit an die RNA: 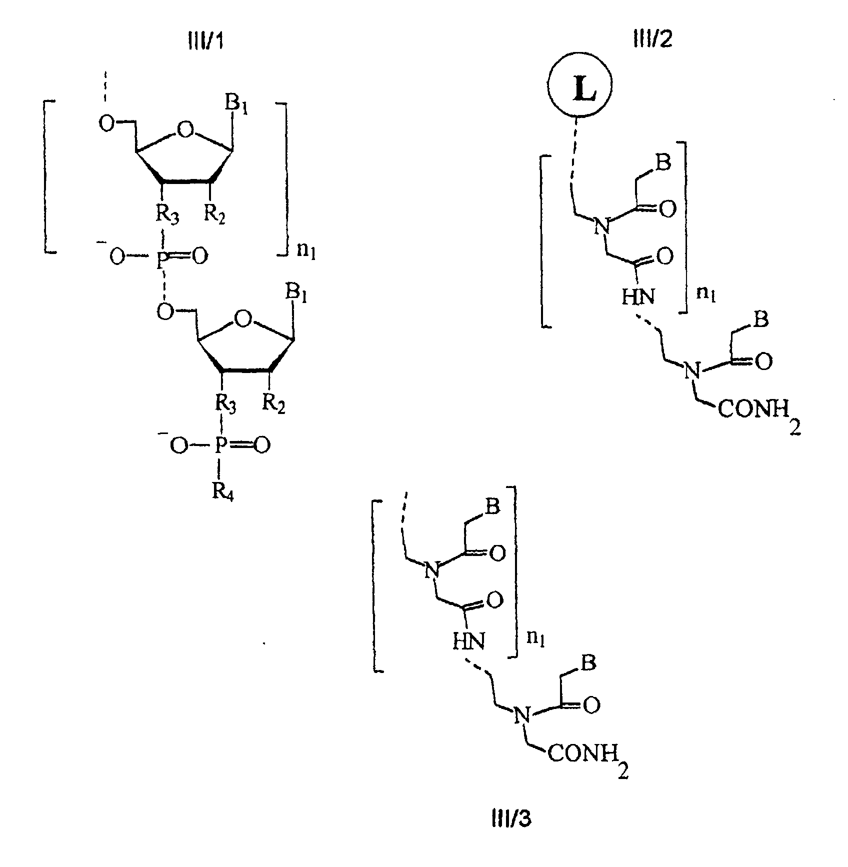 in der
n₁ > 3 ; ≤ 17
B₁ = Thymin, Cytosin, Adenin, Guanin, 5-Propyluracil, 5-Propylcytosin;
R₂= H, F, NH₂, O-Alkyl (C₁-C₅), O-Allyl, O-Methoxyethoxy,
R₃ = NH, O, mit der Maßgabe, daß, wenn R₃= NH, R₂= nicht NH₂, O-Alkyl (C₁-C₅), O-Allyl, O-Methoxyethoxy ist,
R₄ = 2', 3'- Didesoxy-3'-fluorguanosin, 2',3'- Didesoxy-3'-azidoguanosin, 2',3'-Didesoxy-3'-aminoguanosin, 2',3'-Epoxyguanoin, Acyclovir, Ganciclovir, 2'-Desoxyadenosin, 2'-Desoxyguanosin, 2'-Desoxycytidin, 2'-Desoxythymidin,
L = -(PO₂)-OCH₂-COH- CH₂-NH- oder -(PO₂)-OCH₂-CH(CH₂COOH)-(CH₂)₄ NHbedeuten.

Die Herstellung von Phosphorthioat- bzw. Phosphoramidat-modifizierten Oligonucleotiden und der PNA erfolgt in Analogie an sich bekannter Verfahren (Chen, J.-K. et al. Nucleic Acids Res. (1995) 23, 2661-2668 und lyer, R.P. et al, J. Org. Chem. (1990) 55, 4693-4699, Die Verknüpfung der Phosphorthioate mit der PNA erfolgt entsprechend bekannter Verfahren ggf. unter Verwendung von Linkem (Uhlmann, E. et al. Ange. Chem. (1996) 18, 2793-2797). Der Einbau der unter R₄ genannten modifizierten Guanosinderivate in die Otigonucleotide erfolgt in der Form ihrer Triphosphate mit der terminalen Transferase.

Die erfindungsgemäßen Oligonucleotide der allgemeinen Formeln I blockieren beide Bindungsorte der Telomer DNA an dem Enzym gleichzeitig und sind somit hochwirksame und hochselektive Hemmstoffe der Telomerase.

Sie werden ggf. in Form von kationischen Liposomen oder anderen geeigneten Transportmitteln in die Tumorzellen gebracht und führen hier nach permanenter Hemmung der Telomerase zu einer kritischen Verkürzung der Telomer DNA und schließlich zum Zelltod.

Die erfindungsgemäßen Chimären Oligonucleotide werden zur Herstellung pharmazeutischer Darreichungsformen verwendet, in dem sie mit in an sich üblichen pharmazeutischen Zusatz- und Hilfs- und Trägerstoffen formuliert werden.

Die so hergestellten Arzneimittel stellen hochwirksame Cancerostatika dar.

Die Erfindung soll nachfolgend an einem Ausführungsbeispiel näher erläutert werden.

### Ausführungsbeispiel

Das Oligonucleotid Nr.8 wurde im 1µ Mol-Maßstab an einem DNA-Synthesizer der Fa. Applied Biosystem, Modell 391, nach den Protokollen des Geräteherstellers unter Nutzung von Cyanoethylphosphoramiditen hergestellt. Die Phosphorthioat-Bindungen wurden mittels Tetraethylthiuramdisulfid gebildet (Lit.: H. Vu and B. L. Hirschbein, Tetrahedron Lett.(1991) 32, 3005-3008). Der entscheidende Schritt in der automatisierten Synthese der Phosphoramidatbindungen des Oligomeren besteht in der Reaktion des 5'-(N,N-Diisopropylamino-2-cyanoethyl)-phosphoramidit-3'-(trityl)amino-2',3'-didesoxythymidin-Monomeren (Lit.:McCurdy, S. N. et al. Tetrahedron Lett. (1997) 2, 207-210) mit dem an der Festphase gebundenen 3'-OH-Nucleotid, bzw. einem 3'-Aminonucleotid der wachsenden Nucleotidkette. Das resultierende Phosphoramidit wurde zum stabilen Phosphoramidat oxidiert. Nach dem Entfernen der Basenschutzgruppen mit Ammoniak wurde das Oligomere mittels denaturierender Gelelektrophorese gereinigt. Das Oligonucleotid Nr. 8 wurde entsalzt (NAP 10, Phamacia) und lyophilisiert.
Zur Bestimmung der Wirksamkeit der beiden chimären Oligonucleotide Nr. 5 und Nr. 8 an der Telomerase wurden Zellen der menschlichen Tumorzellinie HL60 lysiert und ein 1000-Zellen-Äqivalent dieses Extraktes in dem von Piatyzek et al. 1995 beschriebenen TRAP -Assay (Telomeric Repeat Amplification Protocol) eingesetzt. Im Prinzip wird dabei ein radioaktiv-markierter Primer durch die Aktivität der Telomerase verlängert und das entstandene Telomeraseprodukt nach PCR-Amplifikation und Gelelektrophorese mittels Phosphorimaging quantitativ nachgewiesen. Die Oligonudeotide Nr.5 und Nr.8 sind in der Lage, die Aktivität der Telomerase stark zu hemmen. Eine 50 % Hemmung der Telomerase-aktivität wird durch Oligonucleotid Nr.5 bei 0.5 nM und durch Oligonucleotid Nr. 8 bei 1 nM.

### Literatur

Autexier, C. et al. TIBS (1996) 21, 387-391.
Bacchetti, S. Sem. Cell Develop. Biol. (1996) 7, 31-39.
Counter, C. M. et al. Proc. Natl. Acad. Sci. USA (1994) 91, 2900-2904.
Counter, C. M. et al. Blood (1995) 85, 2315-2320.
Feng, J. et al. Science (1995) 269, 1236-1241.
McCurdy, S. N. et al. Tetrahedron Lett. (1997) 2, 207-210.
Hamilton, S. et al. Chemistry & Biology (1996) 3, 863-867.
Hanvey, J. C. et al, Science (1992) 258, 1481-1485.
Harley, C. B. et al. Mutat. Res. (1991) 256, 271-282.
Healy, K.C. Oncology Res. (1995) 7, 121-130.
Hiyama, E. et al. Cancer Res. (1997) 57, 326-331.
Holt, S. E. et al. Nature Biotechnology (1996) 14, 836-839.
lyer, R. P. et al. J. Org. Chem. (1990) 55, 4693-4699.
Kim, N. W. et al. Science (1994) 266, 2011-2015.
Piatyszek, M. A. et al. Methods in Cell Science (1995) 17, 1-15.
Shay, J. W. et al. Curr Opin. Oncol. (1996) 8, 66-71.
Strahl, C. et al. Mol. Cell. Biology (1996) 16, 53-65.

## Patentansprüche

1. Chimäre Oligonucleotide der allgemeinen Formel I mit einem Phosphorthioat modifizierten Oligonucleotid beliebiger Sequenz am 5'-Ende und einem anderweitig modifizierten Oligomer am 3'-Ende, deren Basensequenz komplementär zur Telomerase RNA ist 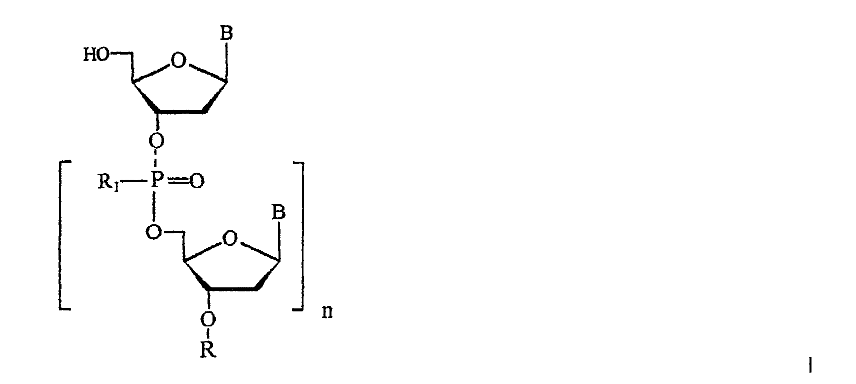 in der
R 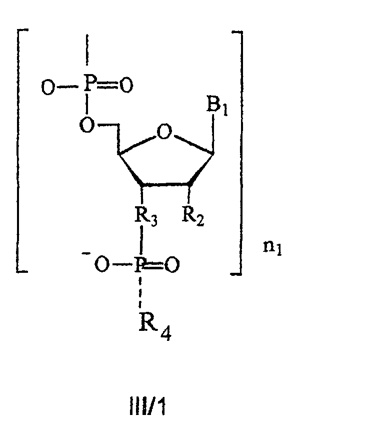 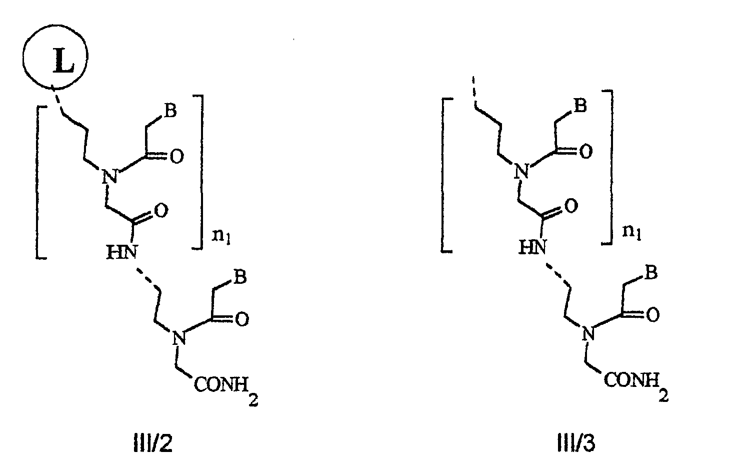 wobei
n > 10; ≤ 20
R₁ = S⁻, CH₃, O⁻
B = Thymin, Cytosin, Adenin, Guanin
n₁ >3 ; ≤ 17
B₁ = Thymin, Cytosin, Adenin, Guanin, 5-Propyluracil, 5-Propylcytosin;
R₂ = H, F, NH₂, O-Alkyl (C₁-C₅), O-Allyl, O-Methoxyethoxy,
R₃ = NH, O, mit der Maßgabe, daß, wenn R₃= NH, R₂= nicht NH₂, O-Alkyl (C₁-C₅), O-Allyl, O-Methoxyethoxy ist,
R₄ = 2',3'- Didesoxy-3'-fluorguanosin, 2',3'- Didesoxy-3'-azidoguanosin, 2',3'-Didesoxy-3'-aminoguanosin, 2',3'-Epoxyguanosin, Acyclovir, Ganciclovir, 2'-Desoxyadenosin, 2'-Desoxyguanosin, 2'-Desoxycytidin, 2'-Desoxythymidin,
L = -(PO₂)-OCH₂-COH- CH₂-NH- oder-(PO₂)-OCH₂-CH(CH₂COOH)-(CH₂)₄-NHbedeuten.

2. Oligonucleotide gemäß Anspruch 1, **dadurch gekennzeichnet, daß** sie Verbindungen der allgemeinen Formel I mit R = Formel III/1 sind

3. Oligonucleotide gemäß Anspruch 1, **dadurch gekennzeichnet, daß** sie Verbindungen der allgemeinen Formel I mit R = Formel III/2 sind

4. Oligonucleotide gemäß Anspruch 1, **dadurch gekennzeichnet, daß** sie Verbindungen der allgemeinen Formel I mit R = Formel III/3 sind

5. Oligonucleotide gemäß Anspruch 1, **dadurch gekennzeichnet, daß** die Sustituenten R₁-R₄ sowie B und B₁ von Nucleotidbaustein zu Nucleotidbaustein verschieden sind

6. Oligonucleotide der Formel I, **gekennzeichnet durch** folgende Nucleotidtreihenfolgen

1.
5´-T*C*A*G*A*T*T*A*G*T*A*C*T*C*G*T*C*A*G*AG TTA GGG TTA G-3´
*= Phosphorthioatverknüpfung
AGTTAGG......Phosphordiesterverknüpfung

2.
5´-T*C*A*G*A*T*T*A*G*G*A*C*T*G*C*T*C*A*G*AG TTA G-3´
*= Phosphorthioatverknüpfung

3.
5´-T*C*A*G*A*T*T*A*G*T*A*C*T*C*G*T*C*A* G*AC AGT TAG  GGT TAG-3´
*= Phosphorthioatverknüpfung

4.
5´-T*C*A* G*A*T* T*A*G* T*A*C* T*C*G* T*C*A* G*AG TTA GAG TTA G-3´
*= Phosphorthioatverknüpfung

5.
5´-T*C*A* *G*A*T* T*A*G* G*A*C* T*G*C* T*C*A* GAₒₘGₒₘ UₒₘUₒₘAₒₘ Gₒₘ-3´
*= Phosphorthioatverknüpfung
ₒₘ =2´-OCH₃- modifizierte Ribose

6.
5´-T*C*A* G*A*T* T*A*G* G*A*C* T*G*C* T*C*A* G*A*Uₒₘ AₒₘGₒₘUₒₘ UₒₘAₒₘGₒₘ -
3´
*= Phosphorthioatverknüpfung
ₒₘ =2´-OCH₃- modifizierte Ribose

7.
5´-T*C*A* *G*A*T* T*A*G* G*A*C* T*G*C* T*C*A* GAGₒₘ UₒₘUₒₘAₒₘ
GₒₘGₒₘGₒₘTₒₘ TₒₘAₒₘGₒₘAₒₘCₒₘAₒₘAₒₘ-3´
*= Phosphorthioatverknüpfung
ₒₘ =2´-OCH₃- modifizierte Ribose

8.
5´-T*C*A* G*A*T* T*A*G* G*A*C* T*G*C GT^amT^am AGG GT^amT^am AGA CAA-3´
*= Phosphorthioatverknüpfung
^am = Phosphoramidatverknüpfung

9.
5´-T*C*A* G*A*T* T*A*G* T*A*C* T*C*G TCA GA-X-TAG GGT TAG ACA A-3´
*= Phosphorthioatverknüpfung
X= 3´-O(PO2)O CH2 CH (CH2COOH-(CH2)4-NH-
TAG GGT TAG ACA A = PNA

10.
5´-T*C*A*G*A*T*T*A*G*T*A*C*T*C*G*T*C*A*G*AG TTA GGG TTAg -3´
*= Phosphorthioatverknüpfung
g= 3´Azidodesoxyguanosin

7. Verwendung von chimären Oligonucleotiden gemäß Anspruch 1 bis 6 zur extrakorparalen Hemmung des Enzyms Telomerase.

8. Verwendung von chimären Oligonucleotiden gemäß Anspruch 1 bis 6 zur Herstellung eines Arzneimittels zur Behandlung von Tumoren.

9. Pharmazeutische Mittel, die als Wirkstoff ein oder mehrere chimäre Oligonucleotide gemäß Anspruch 1 bis 6 enthalten.

10. Pharmazeutische Mittel nach Anspruch 9, dadurch gekehnzeichnet, daß es Cancerostatika sind.

## Claims

1. Chimeric oligonucleotides of general formula I with a phosphor thioate modified oligonucleotide of an arbitrary sequence at the 5' end and a differently modified oligomer at the 3' end, the base sequence of which is complementary to the telomerase RNA 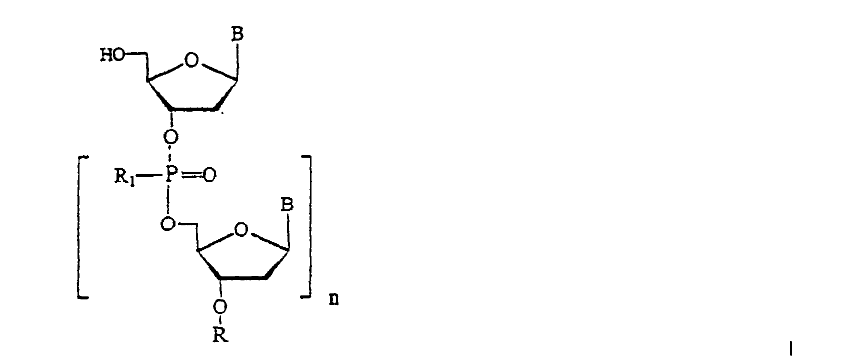 in which R 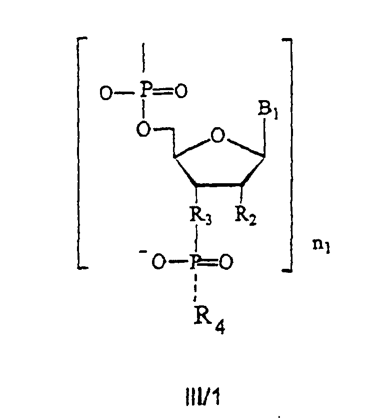 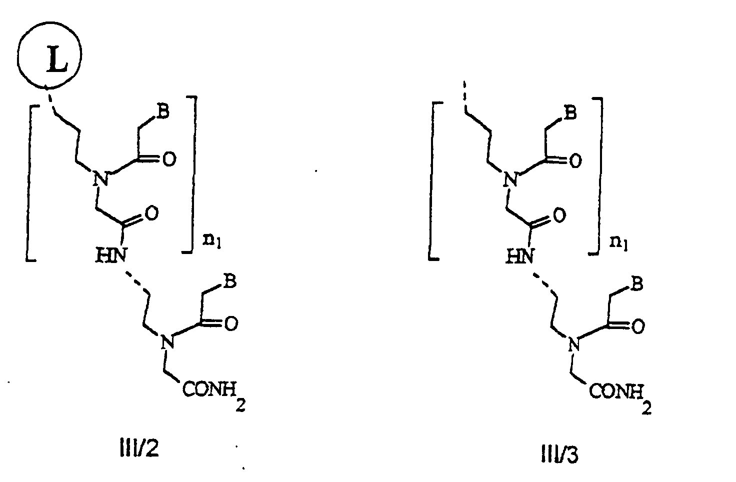 with the meanings:
n > 10; ≤ 20
R₁ = S-, CH₃, O-
B = thymine, cytosine, adenine, guanine
n₁ > 3; ≤ 17
B₁ = thymine, cytosine, adenine, guanine 5-propyluracil, 5-propylcytosine;
R₂ = H, F, NH₂, O-alkyl (C₁-C₅), O-allyl, O-methoxyethoxy,
R₃ = NH, O under the condition that if R₃ = NH, R₂ is not = NH₂, O-alkyl (C₁-C₅), O-allyl, O-methoxyethoxy
R₄ = 2',3'-didesoxy-3'-fluoroguanosine, 2',3'-didesoxy-3'-azidoguanosine, 2',3'-didesoxy-3'-aminoguanosine, 2',3'-epoxyguanosine, acyclovir, ganciclovir, 2'-desoxyadenosine, 2'-desoxyguanosine, 2'-desoxycytidine, 2'-desoxythymidine,
L = (PO₂)-OCH₂-COH- CH₂-NH- or (PO₂)-OCH₂-CH(CH₂COOH)-(CH₂)₄-NH-.

2. Oligonucleotides according to Claim 1, wherein they are compounds of general formula I with R = Formula III/1.

3. Oligonucleotides according to Claim 1, wherein they are compounds of general formula I with R = Formula III/2.

4. Oligonucleotides according to Claim 1, wherein they are compounds of general formula I with R = Formula III/3.

5. Oligonucleotides according to Claim 1, wherein the substituents R₁-R₄ and also B and B₁ differ from one nucleotide component to the next.

6. Oligonucleotides of Formula I, wherein there exist the following nucleotide sequences:

1.
5'-T*C*A*G*A*T*T*A*G*T*A*C*T*C*G*T*C*A*G*AG TTA GGG TTA G-3'
* = phosphor thioate compound
AGTTAGG ... phosphor diester compound

2.
5'-T*C*A*G*A*T*T*A*G*G*A*C*T*G*C*T*C*A*G*AG TTA G-3'
* = phosphor thioate compound

3.
5'-T*C*A*G*A*T*T*A*G*T*A*C*T*C*G*T*C*A* G*AC AGT TAG GGT TAG-3'
* = phosphor thioate compound

4.
5'-T*C*A G*A*T* T*A*G* T*A*C* T*C*G T*C*A* G*AG TTA GAG TTA G-3'
* = phosphor thioate compound

5.
5'-T*C*A* *G*A*T* T*A*G* G*A*C* T*G*C T*C*A GAₒₘGₒₘUₒₘUₒₘAₒₘ Gₒₘ-3'
* = phosphor thioate compound
ₒₘ = 2'-OCH₃ modified ribose

6.
5'-T*C*A* G*A*T* T*A*G* G*A*C* T*G*C* T*C*A* G*A*Uom AomGomUomUomAomGom-3'
* = phosphor thioate compound
om = 2'-OCH3 modified ribose

7.
5'-T*C*A* *G*A*T* T*A*G G*A*C* T*G*C* T*C*A* GAGomUomUomAom GomGomGomTom
TomAomGomAomComAomAom-3'
* = phosphor thioate compound
om = 2'-OCH3 modified ribose

8.
5'-T*C*A* G*A*T* T*A*G* G*A*C* T*G*C GT^amT^am AGG GT^amT^am AGA CAA-3'
* = phosphor thioate compound
^am = phosphor amidate compound

9.
5'-T*C*A* G*A*T* T*A*G* T*A*C T*C*G TCA GA-X-TAG GGT TAG ACA A-3'
* = phosphor thioate compound
X=3'-O(PO2)O CH2 CH (CH2COOH-(CH2)4-NH-
TAG GGT TAG ACA A = PNA

10.
5'-T*C*A*G*A*T*T*A*G*T*A*C*T*C*G*T*C*A*G*AG TTA GGG TTAg-3'
* = phosphor thioate compound
g = 3'azidodesoxyguanosine

7. Use of chimeric oligonucleotides according to Claims 1 to 6 for extra-corporal inhibition of the telomerase enzyme.

8. Use of chimeric oligonucleotides according to Claims 1 to 6 for production of a medication for the treatment of tumours.

9. Pharmaceutical agents containing one or more chimeric oligonucleotides according to Claims 1 to 6 as an active agent.

10. Pharmaceutical agents according to Claim 9 wherein they are cancerostatics.

## Revendications

1. Oligonucléotides chimériques de formule générale I avec un oligonucléotide modifié au phosphoro-thioate de séquence quelconque à l'extrémité 5' et un oligomère modifié autrement à l'extrémité 3' dont la séquence de base est complémentaire à la télomérase ARN. 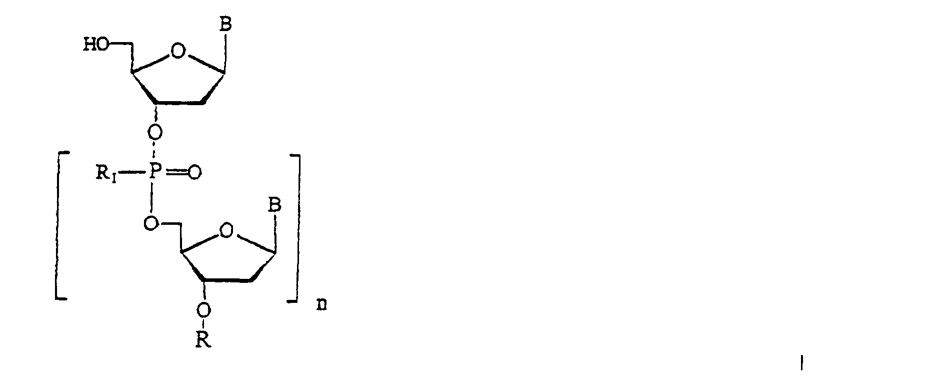 dans laquelle
R 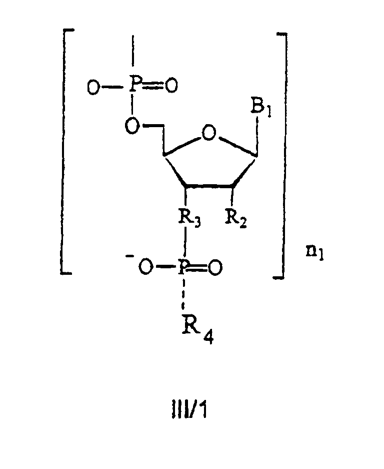 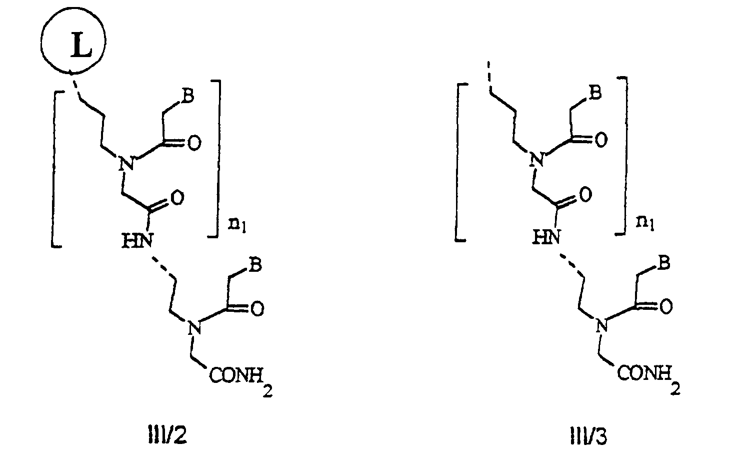 les symboles suivants signifiant
n> 10; ≤ 20
R₁ = S⁻, CH₃, O⁻
B = Thymine, cytosine, adénine, guanine
n₁ > 3; ≤ 17
B₁ = Thymine, cytosine, adénine, guanine, 5-propyluracile, 5-propylcytonsine;
R₂ = H, F, NH₂, O-alcoyle (C₁-C₅), O-allyle, O-méthoxyéthoxy,
R₃ = NH, O, à condition que si R₃ = NH, R₂ = n'est pas NH₂, O-alcoyle (C₁-C₅), O-allyle, O-méthoxyéthoxy,
R₄ = 2', 3'-didesoxy-3'-fluorguanosine, 2', 3'-didesoxy-3'-acidoguanosine, 2', 3'-didesoxy-3'-aminoguanosine, 2', 3'-epoxyguanosine, Acyclovir, Ganciclovir, 2'-desoxyadénosine, 2'-desoxyguanosine, 2'-desoxycytidine, 2'-desoxythymidine,
L = -(PO₂)-OCH₂-COH-CH₂-NH- ou -(PO₂)-OCH₂-CH(CH₂COOH)-(CH₂)₄-NH-.

2. Oligonucléotides conformément à la revendication 1, se caractérisant par le fait qu'ils sont des composés de la formule générale I avec R = Formule III/1

3. Oligonucléotides conformément à la revendication 1, se caractérisant par le fait qu'ils sont des composés de la formule générale I avec R = Formule III/2

4. Oligonucléotides conformément à la revendication 1, se caractérisant par le fait qu'ils sont des composés de la formule générale I avec R = Formule III/3

5. Oligonucléotides conformément à la revendication 1, se caractérisant par le fait que les substituants R₁-R₄ ainsi que B et B₁ sont différents d'élément nucléotide à élément nucléotide.

6. Oligonucléotides de la formule I, se caractérisant par les successions de nucléotides suivants

1.
5'-T*C*A*G*A*T*T*A*G*T*A*C*T*C*G*T*C*A*G*AG TTA GGG TTA G-3'
*= liaison phosphoro-thioate
AGTTAGG..... liaison de diester de phosphore

2.
5'-T*C*A*G*A*T*T*A*G*G*A*C*T*G*C*T*C*A*G*AG TTA G-3'
*= liaison phosphoro-thioate

3.
5'-T*C*A*G*A*T*T*A*G*T*A*C*T*C*G*T*C*A*G*AC AGT TAG GGT TAG-3'
*= liaison phosphoro-thioate

4.
5'-T*C*A*G*A*T*T*A*G*T*A*C*T*C*G*T*C*A*G*AG TTA GAG TTA G-3'
*= liaison phosphoro-thioate

5.
5'-T*C*A**G*A*T*T*A*G*G*A*C*T*G*C*T*C*A*GAₒₘ Gₒₘ Uₒₘ Uₒₘ Aₒₘ Gₒₘ₋3'
*= liaison phosphoro-thioate
ₒₘ= 2'-OCH₃-ribose modifiée

6.
5'-T*C*A*G*A*T*T*A*G*G*A*C*T*G*C*T*C*A*G*A*Uₒₘ Aₒₘ Gₒₘ Uₒₘ Uₒₘ Aₒₘ Gₒₘ-
3'
*= liaison phosphoro-thioate
ₒₘ= 2'-OCH₃-ribose modifiée

7.
5'-T*C*A**G*A*T*T*A*G*G*A*C*T*G*C*T*C*A* GAGₒₘ Uₒₘ Uₒₘ Aₒₘ Gₒₘ Gₒₘ Gₒₘ
Tₒₘ Tₒₘ Aₒₘ Gₒₘ Aₒₘ Cₒₘ Aₒₘ Aₒₘ-3'
*= liaison phosphoro-thioate
ₒₘ= 2'-OCH₃-ribose modifiée

8.
5'-T*C*A*G*A*T*T*A*G*G*A*C*T*G*C GT^amT^am AGG GT^amT^am AGA CAA-3'
*= liaison phosphoro-thioate
^am = liaison phosphoramidate

9.
5'-T*C*A*G*A*T*T*A*G*T*A*C*T*C*G*TCA GA-X-TAG GGT TAG ACA A-3'
*= liaison phosphoro-thioate
X = 3'-O(PO2)O CH2 CH(CH2COOH-(CH2)4-NH-
TAG GGT TAG ACA A = PNA

10.
5'-T*C*A*G*A*T*T*A*G*T*A*C*T*C*G*T*C*A*G*AG TTA GGG TTAg-3'
*= liaison de phosphoro-thioate
g = 3'-acidodesoxyguanosine

7. Emploi d'oligonucléotides chimériques conformément à la revendication 1 à 6 pour l'inhibition extracorporelle de l'enzyme télomérase.

8. Emploi d'oligonucléotides chimériques conformément à la revendication 1 à 6 destiné à la fabrication d'un médicament pour traiter les tumeurs.

9. Produits pharmaceutiques qui contiennent en tant que principe actif un ou plusieurs oligonucléotides chimériques conformément à la revendication 1 à 6.

10. Produits pharmaceutiques conformément à la revendication 9 se caractérisant par le fait qu'ils sont des cancérostatiques.
